# EUROPÄISCHE PATENTSCHRIFT

(11) **EP 1 357 864 B1**
(45) Veröffentlichungstag und Bekanntmachung des Hinweises auf die Patenterteilung: **22.11.2006**
(21) Anmeldenummer: 02785293.8
(22) Anmeldetag: 24.10.2002
(51) Int. Cl.: A61F 2/36

(54) **SCHENKELHALSENDOPROTHESE FÜR EIN KÜNSTLICHES HÜFTGELENK**
FEMORAL NECK ENDOPROSTHESIS FOR AN ARTIFICIAL HIP JOINT
ENDOPROTHESE DU COL DU FEMUR POUR ARTICULATION DE LA HANCHE ARTIFICIELLE

(30) Priorität: 08.11.2001 DE 10154970
(43) Veröffentlichungstag der Anmeldung: 05.11.2003
(73) Patentinhaber: ESKA Implants GmbH & Co. KG, 23556 Lübeck (DE)
(72) Erfinder: GRUNDEI, Hans, 23558 Lübeck (DE)
(74) Vertreter: Fuchs Mehler Weiss & Fritzsche
(86) Internationale Anmeldenummer: PCT/EP2002/011889
(87) Internationale Veröffentlichungsnummer: WO 2003/039411

(56) Entgegenhaltungen:
- EP-A- 0 634 154
- EP-A- 0 679 375
- EP-A- 0 791 342
- EP-A- 0 878 176
- EP-A- 1 205 163
- WO-A-89/11837
- DE-A- 2 724 234
- DE-A- 3 334 058
- DE-A- 19 841 612
- DE-C- 837 294
- DE-U- 20 207 913
- DE-U- 29 921 577
- FR-A- 2 626 169
- FR-A- 2 674 122
- US-A- 4 005 495
- US-A- 5 755 793

## Beschreibung

Es wird eine Schenkelhalsendoprothese für ein künstliches Hüftgelenk beschrieben. Die Merkmale des Oberbegriffs des Anspruchs 1 sind aus der DE-U-29921577 bekannt.

Zur Behandlung eines distruierten, d.h. zerstörten Hüftgelenkkopf, woraus erhebliche Schmerzempfindungen und gravierende funktionelle Einschränkungen des Gelenks resultieren, haben sich im wesentlichen zwei Therapiemöglichkeiten während der letzten Jahrzehnte durchgesetzt:

Zum einen ist es möglich, den Patienten mit einer Orthese zu versorgen, d.h. also mit einem externen Stützapparat. Neben der Tatsache, daß diese Versorgungsmöglichkeit kosmetisch völlig unakzeptable ist, ist sie auch versorgungstechnisch nur suboptimal.

Als weitere, weitverbreitete Möglichkeit ist es vorgesehen, den Patienten mit einer Endoprothese zu versorgen, namentlich mit einer Hüftstielendoprothese, bei welcher ein Stiel in den zuvor freigeräumten Knochenmarksraum des Femurs eingesetzt wird und dort zementlos oder unter Verwendung eines Zements fixiert wird. An der proximalen Seite weist eine derartige Endoprothese dann die Möglichkeit des Anschlusses einer künstlichen Gelenkugel auf.

Gerade die letzte Möglichkeit ist stark in der Patentliteratur vertreten. An dieser Stelle sei nur beispielsweise auf die DE 94 12 408 U1 hingewiesen.

Unter den einen oder anderen der in der Patentliteratur vorgeschlagenen Hüftstielendoprothesen gibt es einige Ansätze, die recht vielversprechend sind im Hinblick auf die Langzeitstabilitiät im Patientenkörper. Früher oder später jedoch, beispielweise nach 10 bis 15 Jahren, ist zu beobachten, daß die Endoprothese einem Verschleiß unterworfen ist, welcher zu der Notwendigkeit führt, in einem Revisionseingriff die implantierte Endoprothese zu entfernen und durch eine neue zu ersetzen. Dies ist freilich nicht ganz unproblematisch, da durch die Resektion und Ausräumung des Knochenmarkraumes ein nicht unerheblicher Anteil natürlichen Knochenmaterials bei der Erstimplantation entfernt worden ist. Das einmal entfernte Material fehlt dann unter Umständen bei einem Revisionseingriff. Dies kommt insbesondere dann zum Tragen, wenn die Patienten relativ jung sind, bei denen also von vornherein davon auszugehen ist, daß sie im Laufe ihres Lebens mindestens einem Revisionseingriff unterworfen werden müssen.

Eine Endoprothese, die mit einer geringfügigeren Resektion natürlichen Knochenmaterials auskommt als die bekannten Hüftstielendoprothesen ist bekannt geworden aus der DE 27 24 234 C2. Das darin beschriebene Prothesenteil wird im oberen Bereich eines Femurs, aber unterhalb des Trochanter minors implantiert, ohne daß ein Stiel in den Knochenmarksraum reichen würde. Diese auch als Druckscheibenprothese bezeichnete Prothese greift im wesentlichen mit einer proximalen Druckplatte über die Kortikalis des resezierten Femurs im Bereich des entfernten Hüfrgelenkkopfes. Sie wird mit einer Druckplatte, die lateral am Femur anliegt, verspannt, derart, daß alle mechanischen Kräfte zwischen der Endoprothese und dem Femur direkt in die Kortikalschicht des Femurs eingeleitet werden, wodurch eine als unzulässig empfundene mechanische Beanspruchung der Spongiosa vermieden werden soll. Lediglich eine geringe Menge von Knochenzement ist für die Arretierung der Druckscheibe im proximalen Bereich notwendig.

Der Philosophie dieser Prothese liegt - wie ausgeführt - die Annahme zugrunde, daß die Spongiosa des Femurs möglichst wenig beansprucht werden soll. Dies wird erkauft durch eine extreme Belastung der Kortikalis des Femurs, indem nämlich sämtliche Kräfte darauf abgelastet und darin eingeleitet werden. Dies entspricht nach heutiger Erkenntnis in keiner Weise den natürlichen Belastungsverhältnissen.

Eine ganz ähnlich wirkende Prothese zeigt im übrigen die WO 89/11837, bei der die Funktion der proximalen Druckplatte gemäß der vorerwähnten Druckschrift wahrgenommen wird durch den speziell ausgebildeten Hüftgelenkkopf, der innenseitig mit einer Ausnehmung versehen ist, an der sich innenseitig der resezierte Femurstumpf unter Druckerzeugung anlegt.

Zwei Prothesen, bei denen augenscheinlich die Hauptlast von der Spongiosa des Femurknochens aufgenommen werden soll, sind bekannt aus der FR 26 26 169 A1 und FR 26 74 122 A1. Bei der erstgenannten Druckschrift der beiden ist vorgesehen, einen proximal einen Steckkonus aufweisenden Gewindebolzen in die Spongiosa einzuschrauben. Dies dürfte innerhalb kürzester Zeit zu schweren Instabilitätsproblem führen. Bei der zweitgenannten Druckschrift der beiden wird eine einen Steckkonus tragende Platte mittels einer Reihe von Knochenschrauben befestigt, wobei die Knochenschrauben in die Spongiosa reichen. Auch hieraus ergeben sich ernsthafte Stabilitätsfragen, da die Spongiosa von Natur aus im Verhältnis zur Kortikalis des Femurknochens punktuell nur gering belastbar ist.

Einen anderen Weg beschreitet die EP-A-0 878 176. In dieser Druckschrift wird eine Schenkelhalsendoprothese beschrieben, die eine zementlos im oberen Bereich eines Femurs unterhalb des Trochanter majors implantierbare Hülse aufweist, die an ihrem proximalen Ende mit einer künstlichen Gelenkkugel versehen ist. Ihre Hülsenaußenseite ist zumindest teilweise mit einer offenmaschigen dreidimensionalen Raumnetzstruktur belegt. Darüber hinaus weist sie ein nach caudal gebogenes, als Stielende ausgebildetes Hülsenende auf, welches ebenfalls zumindest teilweise mit einer offenmaschigen dreidimensionalen Struktur belegt ist. Eine caudal ausgerichtete Stufe in der Hülse im Übergangsbereich zum Hülsenende sorgt für eine Ablastung der eingeleiteten Kräfte in die das Implantat umgebende Spongiosa. Im Gegensatz zu langstieligen Hüftlangstielendoprothesen findet die Endoprothese der vorerwähnten Art in der Metaphyse des Femurknochens Platz. Gegenüber den Langstielendoprothesen sind nur relativ geringe Resektionen von natürlichem Knochenmaterial notwendig, weswegen sie sich den natürlichen Verhältnissen erheblich besser anpaßt als jene der vorerwähnten Druckschriften. Hierzu trägt auch bei, daß die Hülse und das Hülsenende zumindest teilweise mit der erwähnten offenmaschigen dreidimensionalen Raumnetzstruktur belegt ist, in welche Knochentrapekel einwachsen und so für eine dauerhafte Fixation der Endoprothese in der Metaphyse sorgen.

Nun gibt es Indikationen, bei denen die Implantation der Endoprothese gemäß der vorerwähnten Druckschrift wenig erfolgsversprechend ist. Dies ist insbesondere dann der Fall, wenn aufgrund anderer Komplikationen nicht mit einem hinreichenden Einwachsen von Knochentrapekeln in die dreidimensionale Raumnetzstruktur zu rechnen ist. Stoffwechselstörungen wie Diabetes seien als nur ein Beispiel genannt. Gleichwohl besteht das Bedürfnis, auch die hiervon betroffenen Patienten mit einer Schenkelhalsendoprothese zu versorgen, um auch ihnen den Vorteil der geringstmöglichen Resektionen bieten zu können.

Vor diesem Hintergrund ist es nun die Aufgabe der vorliegenden Erfindung, eine Schenkelhalsendoprothese der vorerwähnten Art so weiterzubilden, daß sie in der Metaphyse des Femurs fixiert werden kann, ohne auf die an sich bewährte Fixationsmöglichkeit durch Einwachsen von Knochenmaterial in die Oberfläche des Implantates zurückzugreifen. Hierbei gilt es zu berücksichtigen, daß grundsätzlich Ausfräsungen im natürlichen Knochen, in welche das Implantat einzementiert werden soll, größer ist als das Implantat selbst, damit dieses von einem Zementmantel in einer Stärke von mindestens 2 mm umgeben werden kann. Das Aushärten des Zementes dauert üblicherweise ca. 10 min., während der das Implantat seine Stellung noch verändern kann. Dies bedeutet ein gewisses Risiko dafür, daß das Implantat nach Aushärten des Zementes eine nicht gewünschte Stellung einnimmt, welche zu Fehlfunktionen des Gelenkes führen können.

Gelöst wird diese Aufgabe durch eine Schenkelhalsendoprothese für ein künstliches Hüftgelenk mit den Merkmalen des Anspruchs 1. Weitere vorteilhafte Ausführungsformen sind in den Unteransprüchen angegeben.

Demgemäß weist die Schenkelhalsendoprothese auf:
- eine im oberen Bereich eines Femurs unterhalb des Trochanter majors implantierbare Hülse, die an ihrem proximalen Ende einen Kragen zur Anlage an Resektionsflächen des Schenkelhalses aufweist und mit deren proximalem Ende ein Adapter in Form eines Doppelkonus zur Aufnahme einer Gelenkkugel verbindbar ist,
- ein nach caudal gebogenes, als massives Stielende ausgebildetes Hülsenende, und
- eine caudal ausgerichtete Stufe in der Hülse im Übergangsbereich zum Hülsenende, wobei
- der Doppelkonus des Adapters zwei Steckkonen, deren Längsachsen sich in einem Winkel α im Bereich von 0 < α < 30° schneiden, sowie an der Basis des Steckkonus, der mit der Hülse verbindbar ist, eine Markierung aufweist, welche die Drehposition des Steckkonus in der Hülse anhand einer an dem Kragen vorgesehenen Skala anzeigt.

Die erfindungsgemäße Schenkelhalsendoprothese wird also mit Zement im Schenkelhals implantiert. Ihre Wirkungsweise ist ähnlich jener der zementlos implantierbaren Schenkelhalsendoprothese der schon erwähnten EP 0 878 176. Allerdings ist die Endoprothese an das zementierte Implantieren adaptiert. Wie schon erwähnt, sind Ausfräsungen in einem Röhrenknochen, in dem eine Endoprothese implantiert werden soll, größer als die Endoprothese selbst.
Nach der Ausfräsung bestehen also Freiheitsgrade für die Lage des Implantates im Knochen. Dies tritt bei dem zementlos zu implantierenden Implantaten nicht auf. Die Freiheitsgrade führen dazu, daß das Implantat in der einen oder anderen Lage fixiert werden kann. Hierbei spielt eine große Erfahrung des Operateurs eine wesentliche Rolle. Das Implantat wird von einem Zementmantel mit einer Mindestdicke von 2 mm umgeben. Entsprechend groß muß die Ausfräsung sein. Es ist einsichtig, daß nach direktem Einsetzen des Implantates und Hinzugabe des Knochenzementes die Lage des Implantates immer noch veränderlich ist, da das Aushärten des Zementes ca. 10 min. dauert. Hier muß also u.U. eine Korrekturmaßnahme erfolgen, die erfindungsgemäß darin besteht, den Steckkonus in der Hülse zu verdrehen.
Dies geschieht kontrolliert anhand der Markierung an der Basis des in der Hülse sitzenden Steckkonus sowie der Skala am Kragen. Mittels Verdrehung des Doppelkonus in der Hülse vermag der Operateur etwaige Fehllagen des Implantates in der Metaphyse des Femurs auszugleichen, so daß das künstliche Hüftgelenk später die volle Funktionsbreite inne hat, ohne Beschwerden seitens des Patienten hervorzurufen.

Gemäß einer bevorzugten Ausführungsform ist vorgesehen, daß das Stielende mit nach caudal orientierten Rippen, die besonders bevorzugt von ventral nach dorsal orientiert sind, versehen ist. Diese Rippen vergrößern im Bereich des massiven Stielendes die Auflagefläche im Zementmantel zwischen Implantat und der Spongiosa und bieten damit einer Verteilung der Belastungskräfte eine größere Fläche.

Gemäß einer weiteren bevorzugten Ausführungsform ist vorgesehen, daß die Hülse einen rotationsunsymmetrischen Querschnitt aufweist. Dies bedeutet eine zusätzliche Sicherheit gegenüber einem Verdrehen der Endoprothese in der Metaphyse des Femurs und damit eine Sicherheit gegen etwaige Fehlstellungen des künstlichen Hüftgelenkes.

Die Rotationsunsymmetrie kann bevorzugt dadurch realisiert werden, daß der Querschnitt der Hülse ovalär ist.

In diesem Falle ist die Hauptachse des ovelären Hülsenquerschnitts besonders bevorzugt dorsal-ventral orientiert.

Alternativ kann der Hülsenquerschnitt trapezförmig oder im wesentlichen dreieckig ausgebildet sein, wobei dann in vorteilhafter Weise die Ecken jeweils verrundet sind.

Die Erfindung wird nachfolgend anhand eines Ausführungsbeispieles näher erläutert.

Hierbei zeigt:
- Figur 1: die Ansicht des Implantates mit aufgesetzter Gelenkkugel,
- Figur 2: ein Implantat gemäß Figur 1 mit dem Doppelkonus in anderer Drehposition,
- Figur 3: das Implantat ohne Adapter in Richtung des Pfeiles III in Figur 1,
- Figur 4: eine Schnittansicht entlang der Linie IV-IV in Figur 1,
- Figur 5: eine Ansicht in Richtung des Pfeiles V in Figur 3 gemäß einer anderen Ausführungsform,
- Figur 6: die Ansicht des Doppelkonus in Richtung des Pfeiles VI in Figur 2, und
- Figur 7: eine Ansicht des Implantates in Richtung des Pfeiles VII in Figur 3.

Nachfolgend bezeichnen gleiche Bezugszeichen dieselben Teile.

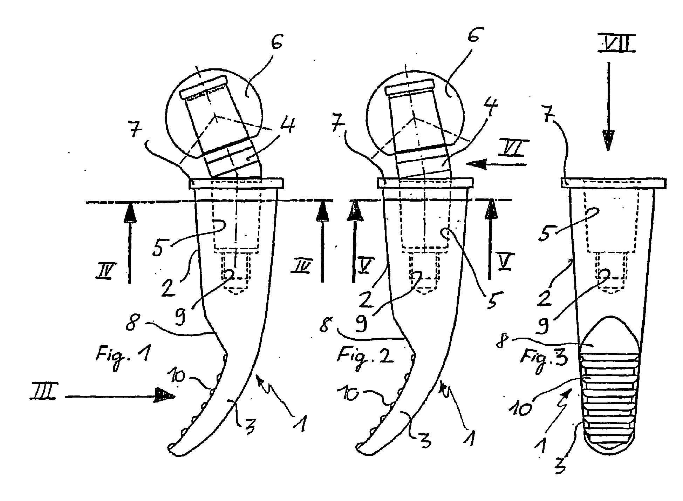

In Figur 1 ist die Schenkelhalsendoprothese allgemein mit dem Bezugszeichen 1 versehen. Diese besteht im wesentlichen aus einer Hülse 2 und dem massiven Stielteil 3. Im Inneren der Hülse 2 ist eine konische Steckhülse 5 ausgeformt, in die ein Steckkonus eines Adapters 4 in Doppelkonusform gesteckt werden kann. Auf dem weiteren Steckkonus des Adapters 4 sitzt eine künstliche Gelenkkugel 6, die dann mit einer künstlichen Beckenpfanne des künstlichen Hüftgelenks zusammenarbeitet. Zwischen der Hülse 2 und dem massiven Stielteil 3 befindet sich in dem Übergangsbereich eine caudal auszurichtende Stufe 8. Diese Stufe 8 dient in erster Linie zu einer Ablastung der Belastungskräfte in der Spongiosa der Metaphyse, in welcher die Stufe 8 nach Implantation liegt.

Proximal ist an die Hülse 2 ein Kragen 7 angeformt, der über den Außendurchmesser der Hülse 2 hinausragt. Der Kragen 7 dient zur Anlage an der Resektionsfläche des Schenkelhalses und einer Begrenzung der Einführtiefe der Endoprothese 1 in den Schenkelhals.

Das Stielende 3 ist vorliegend mit Rippen 10 versehen, die vorliegend von ventral nach dorsal ausgerichtet sind. Die Rippen 10 vergrößern die Ablastfläche der gesamten Endoprothese, und vor allem lateral an der kortikalisierten Seite der natürlichen Spongiosa.

Am Boden der Steckhülse 5 ist eine Gewindebohrung 9 eingelassen. Diese dient dazu, im Revisionsfall ein Werkzeug aufzunehmen, mittels dessen Hilfe die Schenkelhalsendoprothese 1 aus dem Schenkelhals herausgezogen werden kann.

Zur Korrektur einer eventuellen Fehllage der Endoprothese 1 im Schenkelhals des Femurs ist der Adapter 4 mit wenigstens einer Markierung 11 versehen, die im vorliegenden Falle durch zwei weitere Markierungen 11a und 11b ergänzt worden ist. Die Markierung 11 repräsentiert gewissermaßen die Nullage, wohingegen die Markierungen 11a und 11b Auslenkungen um beispielsweise 5° repräsentieren.

Diese Markierungen 11, 11a und 11b arbeiten zusammen mit einer Skala 12 (Figur 7), welche am Kragen 7 vorgesehen ist. Die Skala besteht aus mehreren Markierungen, von welchen jede eine Auslenkung der Drehposition des Adapters 4 aus der Position 0° um jeweils 10° repräsentiert.

Zunächst wird der Operateur den Adapter 4 noch relativ lose in die Steckhülse 5 stecken, und zwar so, daß die Markierung 11 mit der Skalenmarkierung 0° der Skala 12 fluchtet. Sodann wird das Implantat in die Ausfräsung im Schenkelhals gesetzt und der Knochenzement wird in den Zwischenraum zwischen Implantat und Spongiosa gespritzt. In dieser Phase kann der Operateuer etwaige Fehlstellungen der Hülse 2 im Schenkelhals dadurch kompensieren, daß er den noch nicht fest in der Steckhülse 5 sitzenden Adapter 4 in die eine oder andere Richtung dreht. Dies ist beim Übergang von Figur 1 zu Figur 2 veranschaulicht. Hier ist der Adapter 4 um einige Grad verdreht worden, so daß sich die Stellung der Gelenkkugel 6 deutlich verändert hat. Dementsprechend ändert sich die Lage der Markierungen 11, 11a und 11b gegenüber den Einteilungen auf der Skala 12.

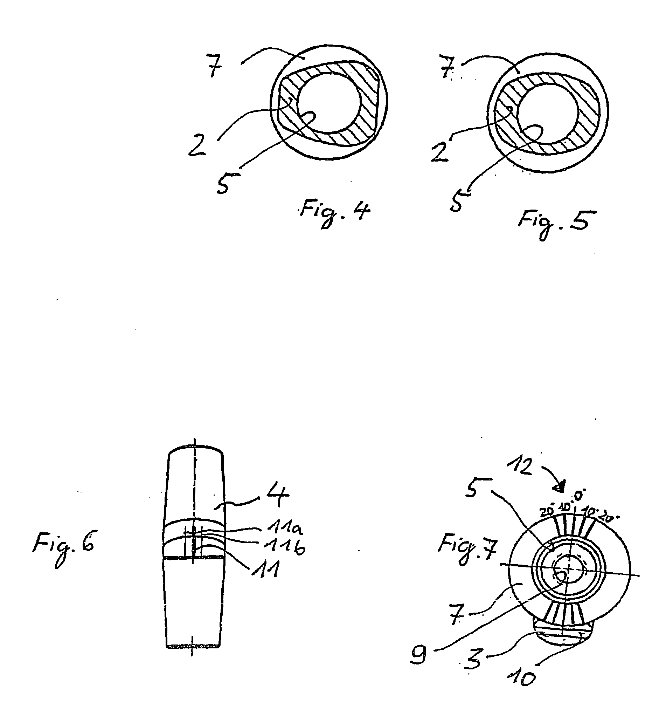

In den Figuren 4 und 5 sind zwei unterschiedliche Ausführungsformen für die Kontur der Hülse 2 abgebildet. Die Figur 4 zeigt eine Schnittansicht entlang der Schnittlinie IV-IV in Figur 1 und Figur 5 eine Ansicht entlang der Schnittlinie V-V in Figur 2.

Figur 4 zeigt einen fast trapezförmigen Querschnitt der Hülse 2. Figur 5 hingegen zeigt eine fast ovaläre Querschnittsfläche der Hülse 2. Hintergrund dieser unterschiedlichen Ausbildungen ist es, die Hülse 2 rotationsunsymmetrisch zu gestalten, damit eine Lageveränderung der Hülse 2 in der Metaphyse des Femurs sicher unterbunden werden kann.

### Bezugszeichen

- 1: Schenkelhalsendoprothese
- 2: Hülse
- 3: Stielteil
- 4: Adapter in Doppelkonusform
- 5: konische Steckhülse
- 6: künstliche Gelenkkugel
- 7: Kragen
- 8: caudal ausgerichtete Stufe
- 9: Gewindebohrung am Boden der Steckhülse
- 10: Rippen
- 11, 11a, 11b: Markierungen auf Doppelkonus
- 12: Skala auf Kragen

## Patentansprüche

1. Schenkelhalsendoprothese (1) für ein künstliches Hüftgelenk, aufweisend
- eine im oberen Bereich eines Femurs unterhalb des Trochanter majors implantierbare Hülse (2), mit deren proximalem Ende ein Adapter (4) in Form eines Doppelkonus zur Aufnahme einer Gelenkkugel (6) verbindbar ist,
- ein nach caudal gebogenes, als massives Stielende (3) ausgebildetes Hülsenende,
- eine caudal ausgerichtete Stufe (8) in der Hülse (2) im Übergangsbereich zum Hülsenende, und
- einen Adapter (4) in Form eines Doppelkonus, welcher zwei Steckkonen aufweist,
**dadurch gekennzeichnet, daß** die Külse (2) an ihrem proximalen Ende einen Kragen (7) zur Anlage an Resektionsflächen des Schenkelhalses aufweist, daß die Längsachsen sich in einem Winkel im Bereich von 0 < α < 30° schneiden, und daß der Doppelkonus an der Basis des Steckkonus, der mit der Hülse (2) verbindbar ist, eine Markierung (11, 11a, 11b) aufweist, welche die Drehposition des Steckkonus in der Hülse (2) anhand einer an dem Kragen (7) vorgesehenen Skala (12) anzeigt.

2. Schenkelhalsendoprothese nach Anspruch 1, bei der das Stielende (3) mit nach caudal orientierten Rippen (10) versehen ist.

3. Schenkelhalsendoprothese nach Anspruch 2, bei der die Rippen (10) von ventral nach dorsal orientiert sind.

4. Schenkelhalsendoprothese nach einem der Ansprüche 1 bis 3, bei der die Hülse (2) einen rotationsunsymmetrischen Querschnitt aufweist.

5. Schenkelhalsendoprothese nach Anspruch 4, bei der der Querschnitt der Hülse (2) ovalär ist.

6. Schenkelhalsendoprothese nach Anspruch 5, bei dem die Hauptachse des ovalären Hülsenquerschnitts dorsal-ventral orientiert ist.

7. Schenkelhalsendoprothese nach Anspruch 4, bei der der Querschnitt der Hülse (2) trapezförmig ist.

8. Schenkelhalsendoprothese nach Anspruch 4, bei der der Querschnitt der Hülse (2) im wesentlichen dreieckig ist.

9. Schenkelhalsendoprothese nach Anspruch 7 oder 8, bei der die Ecken des Querschnitts verrundet sind.

## Claims

1. Femoral neck endoprosthesis (1) for an artificial hip joint, having
- a sleeve (2) which can be implanted in the upper region of a femur below the trochanter major, with the proximal end of which an adapter (4) in the form of a double cone can be connected to receive a joint head (6),
- a caudally bent sleeve end designed as a solid stem end (3),
- a caudally aligned step (8) in the sleeve (2) in the transition region to the sleeve end, and
- an adapter (4) in the form of a double cone, which has two plug-in cones, **characterised in that** the sleeve (2) has at its proximal end, a collar (7) for resting on resection surfaces of the femoral neck, **in that** the longitudinal axes intersect at an angle in the range from 0 < a < 30°, and **in that** the double cone on the base of the plug-in cone, which can be connected to the sleeve (2), has a marking (11, 11a, 11b), which indicates the position of rotation of the plug-in cone in the sleeve (2) using a scale (12) provided on the collar (7).

2. Femoral neck endoprosthesis according to claim 1, in which the stem end (3) is provided with caudally orientated ribs (10).

3. Femoral neck endoprosthesis according to claim 2, in which the ribs (10) are orientated ventrally to dorsally.

4. Femoral neck endoprosthesis according to one of claims 1 to 3, in which the sleeve (2) has a rotationally non-symmetrical cross-section.

5. Femoral neck endoprosthesis according to claim 4, in which the cross-section of the sleeve (2) is oval.

6. Femoral neck endoprosthesis according to claim 5, in which the main axis of the oval sleeve cross-section is orientated dorsally-ventrally.

7. Femoral neck endoprosthesis according to claim 4, in which the cross-section of the sleeve (2) is trapezoidal.

8. Femoral neck endoprosthesis according to claim 4, in which the cross-section of the sleeve (2) is essentially triangular.

9. Femoral neck endoprosthesis according to claim 7 or 8, in which the corners of the cross-section are rounded.

## Revendications

1. Endoprothèse (1) du col du fémur pour une articulation de hanche artificielle, présentant
- une douille (2) que l'on peut implanter dans la zone supérieure d'un fémur en dessous du grand trochanter, à l'extrémité proximale de laquelle on peut raccorder un adaptateur (4) de forme biconique pour recevoir une sphère d'articulation (6),
- une extrémité de douille recourbée en direction caudale se présentant sous la forme d'une extrémité de tige massive (3),
- un palier (8) orienté en direction caudale dans la douille (2) dans la zone de transition vers l'extrémité de la douille, et
- un adaptateur (4) de forme biconique présentant deux cônes de fixation, **caractérisé en ce que** la douille (2) présente à son extrémité proximale un collet (7) pour s'appuyer sur des surfaces de résection du col du fémur, **en ce que** les axes longitudinaux se coupent selon un angle dans la plage 0 < α < 30° et **en ce que** le bicône présente à la base du cône de fixation qui peut être raccordé avec la douille (2), un marquage (11, 11a, 11b) indiquant la position de rotation du cône de fixation dans la douille (2) par le biais d'une échelle (12) prévue sur le collet (7).

2. Endoprothèse du col du fémur selon la revendication 1, dans laquelle l'extrémité de tige (3) est pourvue de nervures (10) orientées en direction caudale.

3. Endoprothèse du col du fémur selon la revendication 2, dans laquelle les nervures (10) sont orientées d'une position ventrale vers une position dorsale.

4. Endoprothèse du col du fémur selon l'une quelconque des revendications 1 à 3, dans laquelle la douille (2) présente une section transversale asymétrique en rotation.

5. Endoprothèse du col du fémur selon la revendication 4, dans laquelle la section transversale de la douille (2) est ovale.

6. Endoprothèse du col du fémur selon la revendication 5, dans laquelle l'axe principal de la section transversale ovale de la douille présente une orientation dorsale-ventrale.

7. Endoprothèse du col du fémur selon la revendication 4, dans laquelle la section transversale de la douille (2) est de forme trapézoïdale.

8. Endoprothèse du col du fémur selon la revendication 4, dans laquelle la section transversale de la douille (2) est sensiblement triangulaire.

9. Endoprothèse du col du fémur selon la revendication 7 ou 8, dans laquelle les angles de la section transversale sont arrondis.
